# EUROPEAN PATENT SPECIFICATION

(11) **EP 2 732 781 B1**
(45) Date of publication and mention of the grant of the patent: **01.07.2015**
(21) Application number: 14154735.6
(22) Date of filing: 03.01.2008
(51) Int. Cl.: A61B 17/34, A61B 1/313, A61B 1/00

(54) **Access sheath with removable optical penetrating member**
ZUGANGSHÜLLE MIT ABNEHMBAREM OPTISCHEM PENETRATIONSELEMENT
GAINE D'ACCÈS ÉQUIPÉE D'UN ÉLÉMENT DE PÉNÉTRATION OPTIQUE AMOVIBLE

(30) Priority: 03.01.2007 US 878483 P
(43) Date of publication of application: 21.05.2014
(62) Divisional of application: 08705502.6
(73) Proprietor: Covidien LP, Mansfield, MA 02048 (US)
(72) Inventor: Smith, Robert, Middletown, CT Connecticut 06457 (US)
(74) Representative: Morgan, Marc

(56) References cited:
- US-A- 5 713 870
- US-A- 5 817 061
- US-A1- 2006 173 479

## Description

### BACKGROUND

### 1. Technical Field

The present disclosure relates to an apparatus for accessing an underlying body cavity and, more particularly, relates to a system incorporating an access sheath having an optical penetrating member which is removable through the access sheath subsequent to accessing the body cavity.

### 2. Background of the Related Art

A variety of surgical procedures require accessing an underlying operative site with a sheath or cannula. For example, in an endoscopic procedure, surgery is performed in a hollow viscus of the body through a small incision or through narrow endoscopic tubes (cannulas) inserted through small entrance wounds in the skin. In laparoscopic procedures, surgery is performed in the interior of the abdomen, typically, through a cannula. Historically, endoscopic surgical procedures were primarily diagnostic in nature. More recently as endoscopic technology has advanced, surgeons are performing increasingly complex and innovative endoscopic surgical procedures.

In a laparoscopic surgical procedure, the abdominal cavity is insufflated with a suitable gas, and a trocar is thereafter utilized to puncture the body cavity. The trocar may include an obturator for penetrating the abdominal tissue and a cannula which is coaxially positioned about the obturator. The obturator is removed from the cannula subsequent to penetration of the tissue thereby leaving the cannula within the tissue for reception of laparoscopic instruments and/or a laparoscope required to perform the desired procedure.

US 2006/0173479 A1 discloses an optical penetrating adapter for surgical portable. US 5,713,870 A1 discloses a retractable safety penetrating instrument.

### SUMMARY

Accordingly, the present disclosure is directed to further improvements in penetrating tissue during surgical procedures such as endoscopic or laparoscopic surgery. In accordance with one embodiment, a system for accessing underlying tissue includes an elongated access sheath defining a longitudinal axis and a penetrating tip releasably mounted to the access sheath and dimensioned for facilitating passage through tissue. The penetrating tip is removable through the access sheath. The penetrating tip may include a transparent region adapted to permit passage of light.

The system may further include an elongated removal member positionable within the access sheath. The elongated removal member may be mounted with respect to the penetrating tip to permit the elongated removal member to remove the penetrating tip through the access sheath subsequent to passage of the penetrating tip through tissue. The elongated removal member is releasably mounted to the penetrating tip. The elongated removal member and the penetrating tip preferably include cooperating structure for effecting releasable coupling of the elongated member to the penetrating tip. The cooperating structure includes one of a bayonet coupling, threaded coupling, tongue and groove coupling and interference coupling.

A sheath housing may be mounted to the access sheath. The sheath housing may include a valve adapted to form a substantial seal about an elongated object passed through the sheath housing.

The system further may include a laparoscope having an illumination system for delivering illuminating light and an imaging system for detecting and transmitting an illuminated image of the surgical object. The laparoscope may be at least partially positioned within the access sheath to permit visualization during advancement of the access sheath and the penetrating tip within the tissue.

### BRIEF DESCRIPTION OF THE DRAWINGS

Preferred embodiments of the present disclosure are described hereinbelow with references to the drawings, wherein:
FIG. 1 is a perspective view of the surgical system for accessing underlying tissue in accordance with the principles of the present disclosure illustrating an access sheath with a releasable optical penetrating tip, an elongated removal instrument for removing the penetrating tip and a laparoscope;
FIG. 2 is an enlarged side view in partial cross-section of the distal end of the access sheath and the optical penetrating tip;
FIG. 3 is a side plan view of an alternate embodiment of the access sheath and the optical penetrating tip;
FIG. 4 is an enlarged side view in partial cross-section of the distal end of the elongated removal instrument and the optical penetrating tip;
FIG. 5 is an enlarged side view in partial cross-section of the distal end of an alternate embodiment of the elongated removal instrument and the optical penetrating tip;
FIG. 6 is an axial plan view of the optical penetrating tip of FIG. 5;
FIG. 7 is a view illustrating the penetrating tip mounted to the access sheath and with the laparoscope positioned therein to permit visualization during penetration of tissue; and

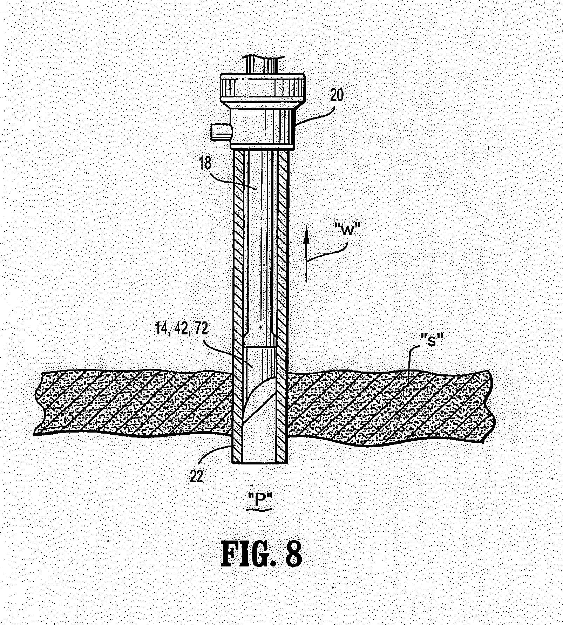

FIG. 8 is a view similar to the view of FIG. 7 illustrating the laparoscope removed from the access sheath and the removal instrument introduced within the access sheath to remove the optical penetrating tip.

### DETAILED DESCRIPTION OF THE PREFERRED EMBODIMENTS

The exemplary embodiments of the surgical system and method of use are discussed in terms of accessing an underlying tissue site, particularly, in accessing an underlying body cavity in connection with an endoscopic procedure. However, it is envisioned that the present disclosure may be employed with a range of surgical applications including surgical, diagnostic and related treatments of diseases, body ailments, of a subject.

In the discussion that follows, the term "proximal" or "trailing" will refer to the portion of a structure that is closer to a clinician, while the term "distal" or "leading" will refer to the portion that is further from the clinician. As used herein, the term "subject" refers to a human patient or other animal. The term "clinician" refers to a doctor, nurse or other care provider and may include support personnel.

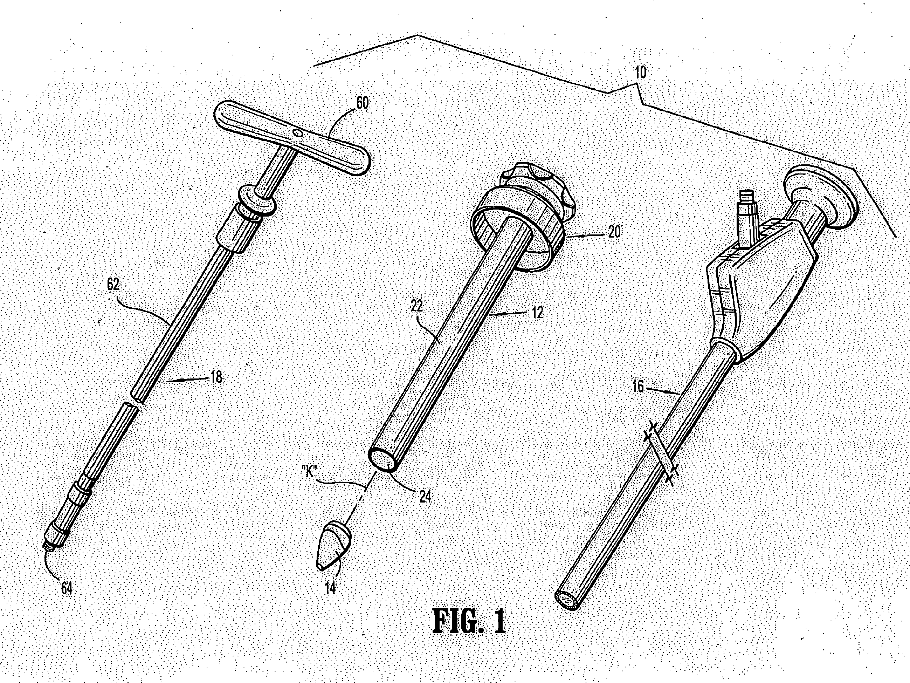

Referring now to the drawings wherein like components are designated by like reference numerals throughout the several views, FIG. 1 illustrates in perspective view, the surgical system 10 in accordance with the principles of the present disclosure. Surgical system 10 includes several components or instruments, namely, access sheath 12 with associated optical penetrating tip 14, laparoscope 16 positionable within the access sheath 12 and removal instrument 18. Access sheath 12 defines a portal for reception of surgical instrumentation such as laparoscope 16, or any variety of laparoscopic or endoscopic instrumentation including clip appliers, stapling instruments, suturing instruments, graspers, forceps, etc. required to perform the desired surgical procedure. In one embodiment, access sheath 12 is a cannula particularly adapted for use in laparoscopic surgery where the peritoneal cavity is insufflated with a suitable gas, e.g., C02, to raise the cavity wall and permit access to the underlying organs. In a conventional laparoscopic procedure, an obturator is positioned within the cannula and utilized to penetrate the abdominal wall. The obturator subsequently is removed from the cannula to permit introduction of the surgical instrumentation utilized to perform the procedure through the passageway.

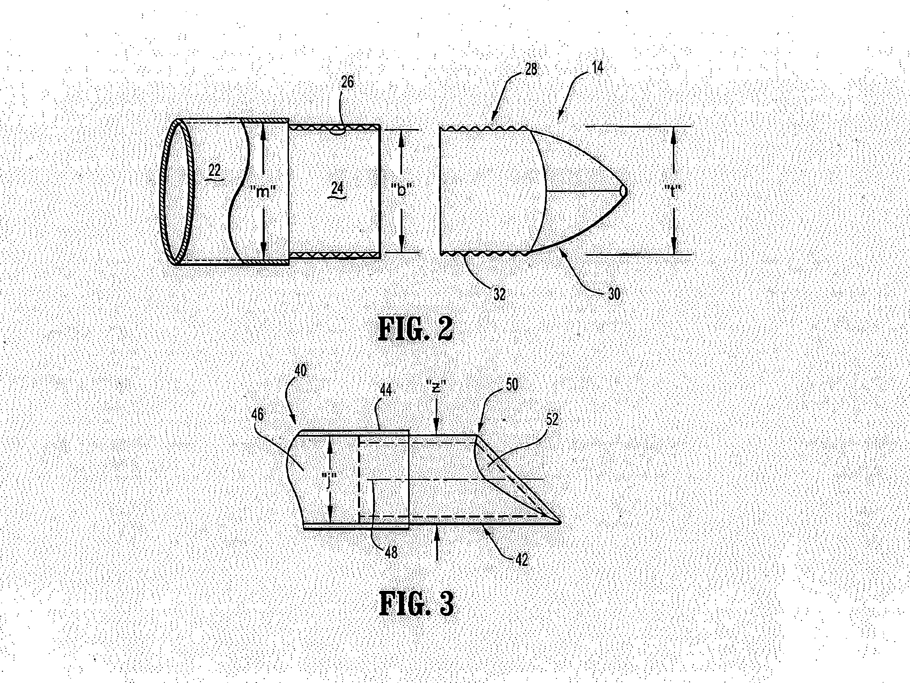

Access sheath or cannula 12 includes access housing 20 and elongated access sleeve 22 extending from the access housing 20. Access sleeve 22 defines a longitudinal axis "k" extending along the length of the sleeve 22. Access sleeve 22 further defines internal longitudinal passage 24 dimensioned to permit passage of surgical instrumentation. Access sleeve 22 may be formed of stainless steel or other rigid materials such as a polymeric material or the like. Sleeve 22 may be clear or opaque. The diameter of sleeve 22 may vary, but typically ranges from about 4.5 to about 15 mm. In one application, access sleeve 22 includes internal threaded portion 26 within leading or distal end of the access sleeve 22 to releasably secure optical penetrating tip 14 to the access sleeve 22. Leading end incorporating threaded portion 26 preferably has a reduced diameter "b" relative to the remainder of the sleeve to define a stepped configuration as best depicted in FIG. 2.

Access housing 20 may include several components connected to each other through conventional means or alternatively may be a single housing component. Access housing 20 may be attached to access sleeve 22 by any suitable means or may be integrally formed with the access sleeve 22. Access housing 20 may further include an internal zero closure valve which is adapted to close in the absence of a surgical instrument and/or in response to the pressurized environment of the insufflation gases present in the abdominal cavity. One suitable zero closure valve contemplated for use with access housing 20 is a duck bill valve, flapper valve, or the like.

Access housing 20 may also include an internal seal preferably adapted to form a substantial fluid tight seal about an instrument inserted through the seal. One suitable internal seal is a flat disc-shaped valve, balloon valve, etc.... The internal seal may comprise a flat disc- shaped, conical, or hourglass-shaped member including a fabric material molded with an elastomer. The seals disclosed in certain embodiments of commonly assigned U.S. Patent No. 6,482,181 may be used. Seals disclosed in certain embodiments of commonly assigned U.S. Patent Application No. 2004/0066008A1, filed October 4, 2002 may be used. In a further alternative, the internal seal is preferably a fabric seal and is desirably arranged so as to have a constriction. For example, the valve may have the general shape of an hourglass. The fabric can be a woven material, a braided material, or a knitted material. The type of material is selected to provide a desired expansiveness. For example, a braid of varying end count and angle may be selected. A preferred material is a synthetic material such as nylon, Kevlar (Trademark of E.I. DuPont de Nemours and Company) or any other material that will expand and compress about an instrument inserted therethrough. The selected material desirably minimizes or prevents the formation of gaps when the instrument is introduced into the seal. The material of the seal may be porous or impermeable to the insufflation gas. If porous, the seal may include a coating of a material which is impermeable to the insufflation gas or at least a portion of the valve may be coated. In addition, the fabric may be coated on its interior with urethane, silicon or other flexible lubricious materials to facilitate passage of an instrument through the seal. In certain embodiments, the fabric is twisted about the axis "a" so as to form a constriction or closed portion. The fabric is desirably constructed of a material and/or arranged so that the fabric forms a constriction or closure. The seal may also be molded so as to have a constriction or may be knitted, braided or woven so as to have a constriction. Other arrangements for the seal are also envisioned.

Referring now to FIGS. 1-2, optical penetrating tip 14 of the present disclosure will be discussed. Optical penetrating tip 14 is contemplated for mounting to access sleeve 22 to provide access sheath 12 with penetrating capabilities thus obviating the need for a separate obturator introduced within the access sleeve 22. Optical penetrating tip 14 when mounted to access sleeve 22 is particularly suitable for use with a viewing device such as an endoscope or laparoscope 16 introduced within access sleeve 22. In this capacity, optical penetrating tip 14 has a transparent region or window to permit direct visualization of body tissue with laparoscope 16 during penetration of the peritoneal cavity or other tissue portions. In one application, optical penetrating tip 14 may have lens surfaces to modify, correct or alter visualization through the optical penetrating tip 14. Optical penetrating tip 14 is dimensioned to pass through body tissue and may incorporate structure to separate, retract, dissect, cut, puncture, or pierce the body tissue. Such structure is inclusive of cutting edges, blades, points, etc.

Optical penetrating tip 14 includes proximal mounting section 28 and distal penetrating section 30. Proximal mounting section 28 is generally cylindrical in configuration defining external thread 32 which cooperates with internal thread 26 within access sleeve 22 to connect the two components. Other means for mounting optical penetrating tip 14 to access sleeve 22 are also envisioned including a bayonet coupling, snap fit, tongue and groove mechanism, etc. Some of these methodologies will be discussed hereinbelow. Optical penetrating tip 14 may comprise a polymeric material and be fabricated via known injection molding techniques. Alternatively, optical penetrating tip 14 may comprise an optical glass. In one embodiment, distal penetrating section 30 defines a transparent region or window which permits visualization along the axis "k" of access sleeve 22 and, desirably, locations offset relative to the axis "k". The term "transparent" is to be interpreted as having the ability to permit the passage of light with or without clear imaging capabilities. Moreover, the transparent material includes any transparent or translucent material or any material which is not opaque to visible light or other radiation utilized for imaging. It is also to be appreciated that only a portion of distal penetrating section 30 needs to be transparent. Furthermore, a portion of optical penetrating tip 14 or the entire tip may be translucent or transparent.

Distal penetrating section 30 including the transparent window may define a variety of geometrical configurations including the generally conically arrangement depicted in FIGS.1-2. Alternatively, distal penetrating section 30 may be pyramidal in configuration having cutting edges, incorporate a dolphin-shaped design or incorporate a cutting blade. The leading end of distal penetrating section 30 may be blunt or pointed. Moreover, distal penetrating section 30 may be intended to penetrate tissue through an incising action or through a more blunt dissecting approach.

Optical penetrating tip 14 preferably defines a maximum outer diameter "t" which is less than the internal diameter of the proximal portion of access sleeve 22 to permit removal of the optical penetrating tip 14 through the access sleeve 22. Specifically, the outer diameter of external thread 32 and the outer diameter of optical penetrating tip 14 is less than the inner diameter "m" of access sleeve 22 to enable the optical penetrating tip 14 to be removed via removal instrument 18 subsequent to penetration of the body cavity. Moreover, the reduction in the diameter of leading end of access sleeve (having diameter "b") and in the diameter "t" of optical penetrating tip enables unencumbered removal of optical penetrating tip 14 through access sheath 12. Removal of optical penetrating tip 14 from access sheath 12 will be discussed in greater detail hereinbelow.

FIG. 3 illustrates an alternate embodiment of access sheath 40 and optical penetrating tip 42. In accordance with this embodiment, leading end 44 of access sleeve 46 of access sheath 40 is devoid of internal threads and may define an internal diameter "j" which is constant throughout its length. Optical penetrating tip 42 has proximal section 48 which is generally cylindrical and is also devoid of threading. The maximum diameter "z" of optical penetrating tip 48 preferably approximates the inner diameter "j" of access sleeve 46 whereby a close tolerance or frictional fit is established between the two components. With this arrangement, optical penetrating tip 42 is releasably mountable to access sheath 40. Also, with this embodiment, transparent window 50 of optical penetrating tip 42 defines a lens structure incorporating several lens surfaces 52 to widen or alter the angle of view of laparoscope 16. For example, transparent window 50 may increase the field of view of laparoscope 16 or, alternatively, may alter the view to provide an inclined angle of view. Other arrangements are also envisioned.

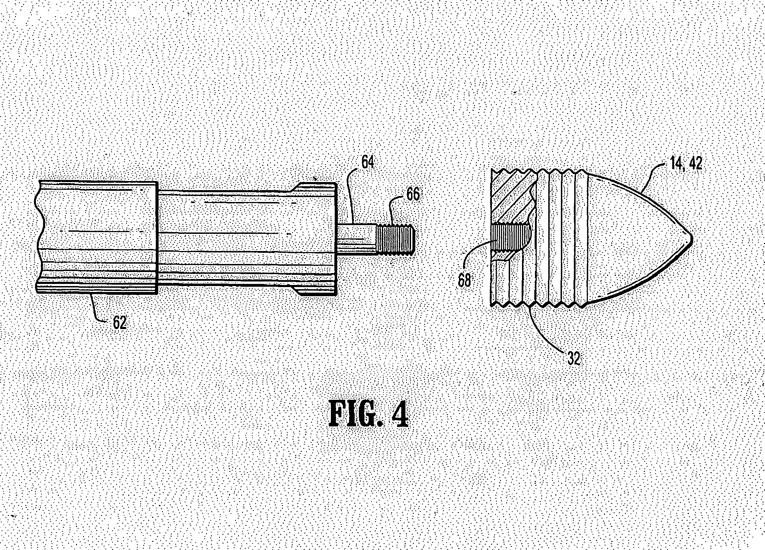

Referring now to FIG. 4, in conjunction with FIG. 1, removal instrument 18 of surgical system 10 will be discussed. Removal instrument 18 includes handle 60 and elongated shaft 62 extending distally from the handle 60. Handle 60 may be "t-shaped" or any other structure appropriately dimensioned for manual engagement by the clinician. Elongated shaft 62 defines leading end 64 having a reduced diameter with external thread 66. External thread 66 cooperates with internal thread 68 of optical penetrating tip 14, 42 to releasably mount removal instrument 18 to the optical penetrating tip 14, 42. In one application, the threads of external thread 66 of removal instrument 18 and the threads of internal thread 68 of optical penetration tip 14 are in the same direction to enable the clinician to connect the removal instrument 18 to the optical penetrating tip 14 and thereafter release the optical penetrating tip 14 from its mounting to access sleeve 22 by rotating handle 60 in the same direction. This also potentially minimizes release of removal instrument 18 from the optical penetrating tip 14. Other means for coupling removal instrument 18 to optical penetrating tip 44 are also envisioned including a bayonet coupling, tongue and groove arrangement, frictional fit, magnetic relationship, vacuum or suction means, hook and loop means or the like.

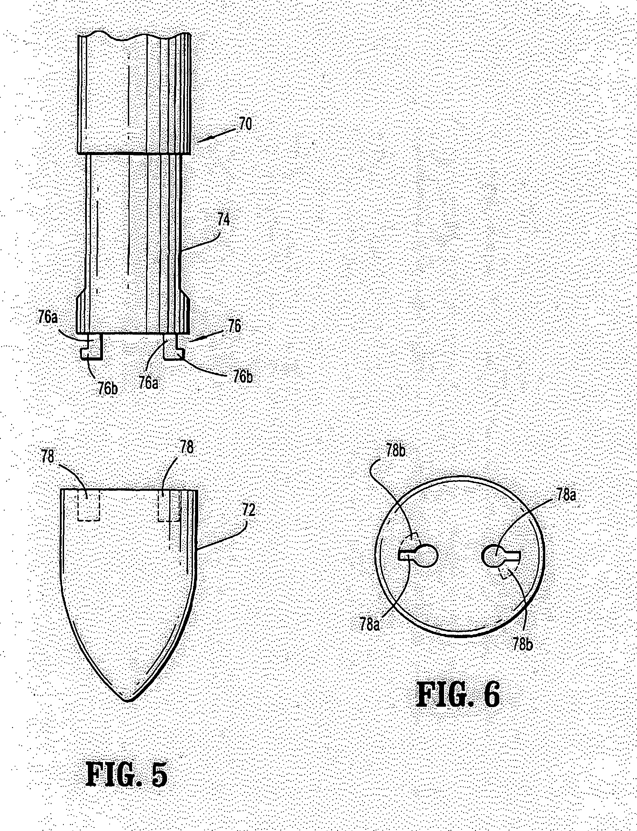

FIGS. 5 and 6 illustrate an alternate embodiment of removal instrument 70 and optical penetrating tip 72. In accordance with this embodiment, removal instrument 70 includes leading end 74 with at least one external pin 76, preferably, two opposed pins 76 extending radially outwardly from the external surface of elongated shaft. External pins 76 are received within corresponding slots 78 within the interior of optical penetrating tip 72 to releasably connect the components through a bayonet coupling relationship. In particular, each pin 76 includes longitudinal pin leg 76a and transverse pin leg 76b. Each slot 78 includes corresponding longitudinal slot portion 78a and transverse slot portions 78b. Pin 76 is received within longitudinal slot portion 78a of slot 78 during initial insertion of leading end 74 within optical penetrating tip 72. Once pins 76 bottom out, removal instrument 70 is rotated whereby transverse pin leg 76b traverses transverse slot portion 78b thereby releasably connecting optical penetrating tip 70 to the removal instrument 70.

Referring again to FIG. 1, laparoscope 16 may be any conventional scope suitable for endoscopic applications including, e.g., a laparoscope, arthroscope, colonoscope, etc. In one preferred embodiment, laparoscope 16 may be the scope disclosed in commonly assigned U.S. Patent No. 5,412,504 to Leiner, the entire contents of which disclosure are hereby incorporated by reference. Laparoscope 16 incorporates an optical train or lens arrangement which is capable of transmitting an image of an object from the distal or objective lens through the eyepiece or monitor for viewing by the surgeon. Further details of laparoscope 16 may be ascertained by reference to the '504 patent.

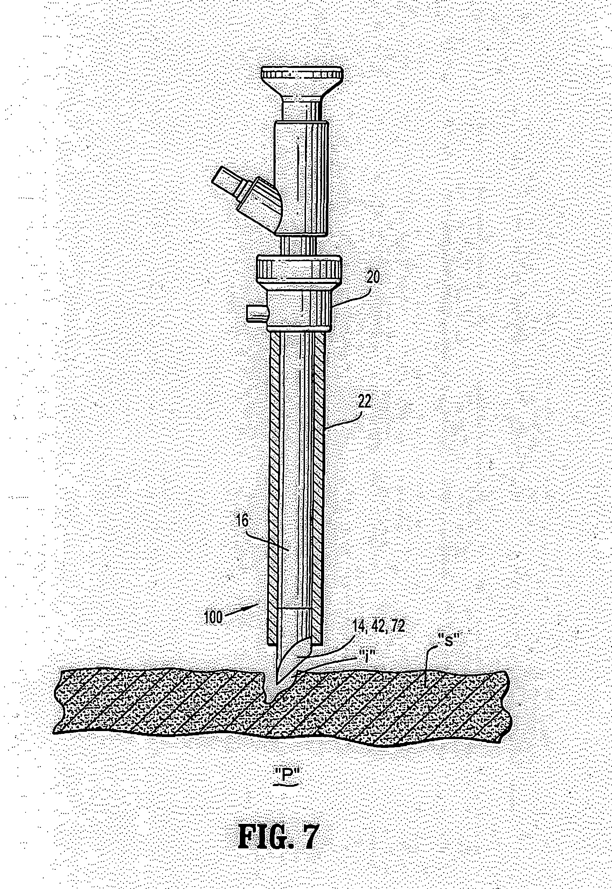

Referring now to FIG. 7, the use of the system 10 during a laparoscopic surgery will be discussed. The peritoneal cavity "p" is insufflated as is conventional to raise the cavity wall to provide greater access to tissue and organs therewithin. Thereafter, any of the aforementioned optical penetrating tips 14,42,72 is mounted to access sleeve 22 of access sheath 12. Laparoscope 16 is positioned within access sheath 22. The internal seal within access housing 20 may form a fluid tight seal about the laparoscope 16. As appreciated, laparoscope 16 is advanced within access sleeve 22 until the distal end of the laparoscope 16 is adjacent the transparent window of optical penetrating tip 14,42,72. In this position, the distal lens element of the laparoscope 16 is adjacent the transparent window so as to be capable of viewing the tissue being entered. Laparoscope 16 may be secured relative to the access sheath 12 utilizing a resilient washer or a cam locking system incorporated with the access sheath 12 or formed separately therefrom.

The procedure is continued by positioning optical penetrating tips 14,42,72 within a previously formed opening or incision "i" in tissue "s" and advancing the optical penetrating tip 14,42,72 to retract, dissect, or penetrate the tissue. Alternatively, optical penetrating tip 14,42,72 may pierce the tissue to form the incision "i". During penetration of the body tissue, the surgeon observes the underlying tissue through the laparoscope 16 to ensure there is no undesired contact with organs, tissue, etc. lying beneath the peritoneal lining. In instances where a video system is utilized, the surgeon simply observes the penetration of body tissue "s" via any known video monitor. Once the surgeon penetrates the body tissue "s" and positions the distal end of access sleeve 22 in the desired position within the peritoneal cavity "p" as observed through the laparoscope 16, the surgeon discontinues the application of force.

Laparoscope 16 may then be removed from access sheath 12. Thereafter, removal instrument 18 is advanced within access sheath 12 and advanced until the leading end is adjacent optical penetrating tip 14,42,72. Removal instrument 18 is then coupled to optical penetrating tip 14,42,72 in any of the aforementioned manners, which is dependent on the coupling structure. Removal instrument 18 is removed from access sheath 12 as shown by the directional arrows "w" to remove optical penetrating tip 14,42,72 leaving access sheath 12 positioned within the body tissue "s'. Endoscopic instrumentation is advanced within access sheath 12 to perform the desired surgery. Surgery is then carried out through other access sheaths or cannula assemblies which access the peritoneal cavity.

It will be understood that various modifications and changes in form and detail may be made to the embodiments of the present disclosure without departing from the scope of the invention. Therefore, the above description should not be construed as limiting the invention but merely as exemplifications of preferred embodiments thereof. Those skilled in the art will envision other modifications within the scope of the present invention as defined by the claims appended hereto. Having thus described the invention with the details and particularity required by the patent laws, what is claimed and desired protected is set forth in the appended claims.

## Claims

1. A system for accessing underlying tissue, which comprises:
an elongated access sheath (12) defining a longitudinal axis;
a penetrating tip (14) releasably mounted to the elongated access sheath and configured to be passed through tissue, the penetrating tip having a transparent region for direct visualization of tissue, the penetrating tip being configured to be removed through the elongated access sheath; and
an elongated removal member (18) configured to be positioned within the elongated access sheath and releasably mountable to the penetrating tip to permit the elongated removal member to remove the penetrating tip through the elongated access sheath subsequent to passage of the penetrating tip through tissue.

2. The system according to claim 1 wherein at least one of the elongated removal member and the penetrating tip includes cooperating structure for effecting releasable coupling of the elongated removal member to the penetrating tip.

3. The system according to claim 2 wherein the cooperating structure includes one of a bayonet coupling, threaded coupling, tongue and groove coupling and interference coupling.

4. The system according to claim 1 including a laparoscope having an illumination system for delivering illuminating light and an imaging system for detecting and transmitting an illuminated image of a surgical object, the laparoscope configured to be positioned at least partially within the access sheath in the absence of the elongated removal member to permit visualization during entry of the access sheath and the penetrating tip within tissue.

5. The system according to claim 1 including a sheath housing mounted to the access sheath.

6. The system according to claim 5 wherein the sheath housing includes a valve adapted to form a substantial seal about an elongated object passed through the sheath housing.

7. The system according to claim 5 wherein the sheath housing includes a zero closure valve.

## Patentansprüche

1. Ein System zum Zugreifen auf ein tiefer liegendes Gewebe, welches umfasst:
eine längliche Zugangshülle (12), die eine Längsachse definiert;
eine Penetrationsspitze (14), die lösbar an der länglichen Zugangshülle angebracht ist und eingerichtet ist, durch Gewebe hindurchzudringen, wobei die Penetrationsspitze einen transparenten Bereich zur direkten Visualisierung von Gewebe hat, wobei die Penetrationsspitze eingerichtet ist, durch die längliche Zugangshülle hindurch entfernt zu werden; und
ein längliches Entfernelement (18) dafür eingerichtet innerhalb der länglichen Zugangshülle positionierbar zu sein und lösbar anbringbar an der Penetrationsspitze, um dem längliches Entfernelement zu ermöglichen, die Penetrationsspitze durch die längliche Zugangshülle zu entfernen, nachdem die Penetrationsspitze durch das Gewebe gedrungen ist.

2. Das System nach Anspruch 1, wobei zumindest eines der Elemente längliches Entfernelement und Penetrationsspitze eine Kooperationsstruktur aufweist, um das lösbare Koppeln des länglichen Entfernelements mit der Penetrationsspitze zu bewerkstelligen.

3. Das System nach Anspruch 2, wobei die Kooperationsstruktur eines der Elemente Bajonettverbindung, Gewindeverbindung, Nut-Feder-Verbindung und Übermaßverbindung umfasst.

4. Das System nach Anspruch 1, aufweisend ein Laparoskop mit einem Beleuchtungssystem zum Bereitstellen von Beleuchtungslicht und mit einem Abbildungssystem zum Detektieren und Übertragen eines beleuchteten Bildes des chirurgischen Objekts, wobei das Laparoskop ausgeführt ist, bei Abwesenheit des länglichen Entfernelements wenigstens teilweise innerhalb der Zugangshülle positionierbar zu sein, um eine Visualisierung während des Eindringens der Zugangshülle und der Penetrationsspitze in das Gewebe zu erlauben.

5. Das System nach Anspruch 1, aufweisend ein Hüllengehäuse, das an der Zugangshülle angebracht ist.

6. Das System nach Anspruch 5, wobei das Hüllengehäuse ein Ventil aufweist, das dafür eingerichtet ist, eine wesentliche Versiegelung um ein längliches Objekt, das durch das Hüllengehäuse hindurchgeführt ist, zu bilden.

7. Das System nach Anspruch 5, wobei das Hüllengehäuse ein Schnellverschlussventil aufweist.

## Revendications

1. Système permettant d'accéder à un tissu sous-jacent, qui comprend :
une gaine d'accès allongée (12) définissant un axe longitudinal ;
une pointe de pénétration (14) montée de manière amovible sur la gaine d'accès allongée et configurée pour être passée à travers le tissu, la pointe de pénétration ayant une région transparente permettant la visualisation directe du tissu, la pointe de pénétration étant configurée pour être retirée à travers la gaine d'accès allongée ; et
un élément de retrait allongé (18) configuré pour être positionné dans la gaine d'accès allongée et montable de manière amovible sur la pointe de pénétration afin de permettre à l'élément de retrait allongé de retirer la pointe de pénétration à travers la gaine d'accès allongée après le passage de la pointe de pénétration à travers le tissu.

2. Système selon la revendication 1, dans lequel au moins l'un(e) ou l'autre de l'élément de retrait allongé et de la pointe de pénétration comprend une structure de coopération pour assurer un couplage amovible de l'élément de retrait allongé avec la pointe de pénétration.

3. Système selon la revendication 2, dans lequel la structure de coopération comprend l'un ou l'autre d'un couplage à baïonnette, d'un couplage à vis, d'un couplage à languette et rainure et d'un couplage par interférence.

4. Système selon la revendication 1, comprenant un laparoscope ayant un système d'éclairage pour fournir de la lumière d'éclairage et un système d'imagerie pour détecter et transmettre une image éclairée d'un objet chirurgical, le laparoscope étant configuré pour être positionné au moins en partie dans la gaine d'accès en l'absence de l'élément de retrait allongé pour permettre une visualisation au cours de l'entrée de la gaine d'accès et de la pointe de pénétration dans le tissu.

5. Système selon la revendication 1, comprenant un logement de gaine monté sur la gaine d'accès.

6. Système selon la revendication 5, dans lequel le logement de gaine comprend une soupape qui est à même de former un joint étanche substantiel autour d'un objet allongé passé à travers le logement de gaine.

7. Système selon la revendication 5, dans lequel le logement de gaine comprend une soupape à fermeture zéro.
